# EUROPEAN PATENT SPECIFICATION

(11) **EP 0 594 950 B1**
(45) Date of publication and mention of the grant of the patent: **27.01.1999**
(21) Application number: 93111735.2
(22) Date of filing: 22.07.1993
(51) Int. Cl.: A61K 39/385, A61K 39/05, A61K 39/08, A61K 39/10, A61K 39/102

(54) **Combination pediatric vaccine with enhanced immunogenicity of each vaccine component**
Pädiatrische Kombinationsvakzine mit verbesserter Immunogenizität jeder Vakzine komponente
Vaccin pédiatrique combiné à immunogénéite augmentée de chaque constituant du vaccin

(30) Priority: 27.10.1992 US 966995
(43) Date of publication of application: 04.05.1994
(73) Proprietor: American Cyanamid Company, Madison, New Jersey 07940-0874 (US)
(72) Inventor: Paradiso, Peter R., Pittsford, New York 14534 (US); Hogerman, Deborah A., Rochester, New York 14609 (US); Madore, Dace Viceps, Pittsford, New York 14534 (US); Hackell, Jill G., New City, New York 10956 (US)
(74) Representative: Talbott, Dawn Jacqueline

(56) References cited:
- EP-A- 0 245 045

## Description

The invention relates to a combination pediatric vaccine comprising diphtheria antigen, tetanus antigen, pertussis antigen and a conjugate of fragments of the capsular polysaccharide antigen of Haemophilus influenzae type b (Hib) and a diphtheria CRM₁₉₇ protein (CRM₁₉₇) in a single immunizing dose. This combination vaccine exhibits improved immunogenicity in infants for each of the four vaccine components when compared to DTP and Haemophilus influenzae type b conjugate (Hib conjugate) vaccines administered concurrently but separately. The invention also relates to the use of a diphtheria antigen, tetanus antigen, pertussis antigen and a conjugate of fragments of the capsular polysaccharide antigen of Haemophilus influenzae type b (Hib) and a diphtheria CRM₁₉₇ protein (CRM₁₉₇) in the preparation of said combined vaccine composition.

Combined vaccines offer many advantages and their use is generally encouraged. The optimal age for vaccination against many diseases is during infancy and early childhood. Typically during this period, therefore, many vaccinations are scheduled during a short period of time. Simultaneous administration may serve to reduce the costs associated with vaccine administration, increase vaccine acceptance rates, and permit early and wide ranging immunization of children.

Examples of vaccine combinations used for vaccination of children are diphtheria and tetanus toxoids with killed whole cells of Bordetella pertussis (DTP); or with acellular pertussis antigens; combination of live measles, mumps and rubella (MMR); three poliovirus serotypes, either live (oral poliovirus vaccine) or inactive (IPV); and a DTP-IPV combination.

Current US immunization practices require vaccination of young infants starting at two months of age with both (1) a diphtheria and tetanus toxoid and pertussis (DTP) vaccine and (2) a Haemophilus influenzae type b conjugate vaccine. Two such commercially available vaccines are Tri-Immunol® (Lederle Laboratories), and HibTITER® (Praxis Biologics, Inc.). Both of these vaccines are recommended for administration at two, four and six months of age with a booster dose at 15-18 months. An additional dose of DTP is recommended at 4-6 years of age. Both of these vaccines, administered separately, have been remarkably successful in reducing the incidence of disease in the united States.

While it would be desirable to combine the DTP and the Haemophilus influenzae type b conjugate into a single vaccine so as to reduce the number of immunizations a child must receive, such combination must have acceptable safety profile and a protective immunogenicity which is not less than that of the vaccines given separately.

### SUMMARY OF THE INVENTION

A combination pediatric vaccine comprising, in a single immunizing dose, a composition of diphtheria antigen, tetanus antigen, pertussis antigen and a conjugate of Haemophilus influenzae type b capsular polysaccharide fragments and CRM₁₉₇ protein in a pharmaceutically acceptable agueous vehicle has been found to exhibit enhanced immunogenicity in infants for each of the four vaccine components. In particular, the combination vaccine of the present invention comprises a sterile mixture of diphtheria toxoid, aluminum phosphate adsorbed, tetanus toxoid, aluminum phosphate adsorbed, inactivated Bordetella Pertussis and a conjugate of Haemophilus influenzae type b capsular polysaccharide fragments and CRM₁₉₇ protein in a pharmaceutically acceptable agueous vehicle. After shaking, the combination vaccine composition of the present invention is a homogeneous white suspension. In addition, the vaccine components are stable and compatible and do not exhibit intercomponent inhibitory activity.

Methods for simultaneous combined vaccination of infants and for enhancing the immunogenicity of the Haemophilus influenzae type b polysaccharide, diphtheria antigen, tetanus antigen and pertussis antigen vaccine components have also been found. These methods comprise providing a single immunizing dose of diphtheria antigen, tetanus antigen, pertussis antigen and a conjugate of Haemophilus influenzae type b capsular polysaccharide fragments and CRM₁₉₇ protein and injecting this single dose into humans, particularly infants.

### DESCRIPTION OF THE PREFERRED EMBODIMENTS

The combination pediatric vaccine composition of the present invention comprises diphtheria, tetanus and pertussis antigens mixed with a conjugate of capsular polysaccharide fragments of Haemophilus influenzae type b covalently bonded to CRM₁₉₇. In a preferred embodiment, the diphtheria antigen is diphtheria toxoid, aluminum phosphate-adsorbed, and the tetanus antigen is tetanus toxoid, aluminum phosphate-adsorbed. These toxoids can be prepared by methods well known in the art. The diphtheria toxoid and tetanus toxoid are derived from Corynebacterium diphtheriae and Clostridium tetani, respectively, which are grown in media according to the method of Mueller and Miller, J. Immunol., 1941, 40:21-32 and J. Immunol., 1947 56:143-147, and are detoxified by use of formaldehyde. The toxoids are refined by the Pillemer alcohol fractionation method, J. Immunol., 1946, 54:213-224, and are diluted with an aqueous solution containing sodium phosphate monobasic, sodium phosphate dibasic, glycine and thimerosal (mercury derivative) as a preservative.

The pertussis antigen is preferably an inactivated whole cell preparation of a strain or combination of strains of Bordetella pertussis. The inactivated whole cell pertussis preparation is prepared by growing Phase I Bordetella pertussis in a modified Cohen-Wheeler broth containing acid hydrolysate of casein. The Bordetella pertussis is inactivated with thimerosal, harvested, and then suspended in an aqueous solution containing potassium phosphate monobasic, sodium phosphate dibasic, sodium chloride, and thimerosal (mercury derivative) as a preservative.

The DTP components can be provided as a mixture. For example, a commercially available preparation of DTP such as that available from Lederle Laboratories under the name Tri-Immunol® can be used for the combination vaccine composition of the present invention.

The Hib conjugate component of the combination vaccine of the present invention comprises fragments of the bacterial capsular polysaccharide of Haemophilus influenzae, type b, covalently bonded to CRM₁₉₇ protein. The fragments are prepared by periodate cleavage of the purified capsular polysaccharide isolated from Haemophilus influenzae type b grown in a chemically defined medium. The protein CRM₁₉₇ is a non-toxic variant of diphtheria toxin isolated from cultures of C.diphteriae strain C7 (Beta 197) grown in a casamino acids and yeast extract based medium. The Haemophilus influenzae type b fragments are coupled to the CRM₁₉₇ protein by covalent attachment. A particularly preferred method for covalently attaching a saccharide to a protein is reductive amination. The preparation of conjugates of Haemophilus influenzae type b and CRM₁₉₇ by reductive amination is described in US patents 4,673,574, 4,761,283 and 4,902,506. The conjugate is purified by diafiltration to remove unreacted protein or polysaccharide fragments, and reagents, and sterilized by filtration. A commercially available Haemophilus influenzae type b conjugate such as that available from Praxis Biologics, Inc. under the name HibTITER® can be used for the combination vaccine composition of the present invention.

The combination vaccine composition of the present invention is prepared by adding a concentrate of Haemophilus influenzae type b conjugate to a DTP preparation when the DTP vaccine is being diluted to final volume. The final immunizing dose, therefore, remains at 0.5ml. Each single dose of 0.5ml of the combination vaccines is formulated to contain a minimum of 7.5 Lf of diphtheria toxoid, 5 Lf of tetanus toxoid (both toxoids induce not less that 2 units of antitoxin per ml in a guinea pig potency test), 10 µg of purified Haemophilus influenzae type b saccharide and approximately 25 µg of CRM₁₉₇ protein. Each 0.5 ml dose of vaccine is formulated to contain less than 16 OPUs of inactivated pertussis cells. The total human immunizing dose (the first three 0.5 ml doses given) contains an estimate of 12 units of pertussis vaccine with an estimate of 4 protective units per single human dose. Each component of the vaccine - diphtheria toxoid, tetanus toxoid, pertussis and Haemophilus influenzae type b conjugate - meets the required potency standards.

As a preservative, thimerosal (mercury derivative) is added to the combination vaccine to a final concentration of 1:10,000. The aluminum content of the final product dose not exceed 0.85 mg per 0.5 ml dose by assay. The residual free formaldehyde content by assay is ≤0.02%.

The combination vaccine containing each of the four vaccine components (D,T,P, and Hib conjugate) is provided as an aqueous composition. After shaking, the vaccine is a homogenous white suspension. Suitable vaccine adjuvants may also be incorporated into the combination vaccine. The vaccine is administered by the intramuscular route of administration.

A four component combination vaccine was prepared as described hereinabove utilizing DTP (Tri-Immuneol®, Lederle Laboratories) and Hib conjugate (HibTITER®, Praxis Biologics).

The enhanced immunogenicity of each of the four vaccine components for the combined has been shown by clinical trial. Multicenter safety and immunogenicity studies were performed in toddlers and infants. In addition, a large scale safety study was performed in infants to assess the occurrence of rare adverse events in a large number of subjects.

For both the toddler and infant safety and immunogenicity studies, subjects were randomized to receive either a single 0.5 ml dose of one of three consistency lots of the four component combined vaccine (D,T,P, and Hib conjugate, combined) or a 0.5 ml dose of each of a DTP and a Hib conjugate vaccine selected from one of three corresponding lots of DTP and Hib conjugate administered concurrently as separate injections (DTP-Hib conjugate, separate) at 18+/-3 months of age or at approximately 2, 4 and 6 months of age. Thus subjects were assigned to one of six treatment cohorts. Acute safety was assessed by parental report of adverse events for 72 hours following each immunization. Immunogenicity was determined by serologic analysis of antibody to Haemophilus influenzae type b polysaccharide (Hib polysaccharide), diphtheria, tetanus and pertussis.

Each study protocol was analyzed independently. A primary analysis of the safety and immunogenicity data concluded that all three consistency lots of the four component combined vaccine were similar for all parameters tested and thus the data was collapsed. This was also the case for the three corresponding lots of DTP and Hib conjugate which were administered concurrently as separate injections.
a. Toddler Safety and Immunogenicity Study
   A total of 215 subjects (combined vaccine n=107, DTP-Hib conjugate, separate n=108) were enrolled into the toddler study protocol and randomized at each of four clinical sites. Demographic characteristics of all subjects were similar.
   Serologic analysis of pre and one month post-immunization serum samples indicated no difference in response to Hib polysaccharide, diphtheria or tetanus (See Table 1). However, the antibody response to pertussis agglutinins was significantly higher in the combined vaccine cohort (142.58) compared to the DTP-Hib conjugate, separate cohort (82.44; p=0.013).
b. Infant Safety and Immunogenicity Study
   A total of 378 subjects (combined vaccine n=189, DTP-Hib conjugate, separate n=189) were enrolled into the infant study protocol and randomized at each of five clinical sites. Demographic characteristics of all subjects were similar.
   The response to Hib polysaccharide after each of three immunizations was higher in the subjects receiving the combined vaccine (See Table 2). After two doses, the geometric mean titer (GMT) of the combined vaccine group was 0.66 µg/ml compared to 0.34 µg/ml in the DTP-Hib conjugate, separate group (p<0.001). The same trend was observed after the third dose, 6.67 µg/ml compared to 4.42 µg/ml for the combined vaccine and the DTP-Hib conjugate, separate vaccines respectively (p=0.034).
   Serologic response to the DTP components was measured one month after the third dose was given. The GMT of antibody to diphtheria (0.71 IU/ml vs. 0.40 IU/ml; p=0.009), tetanus (8.20 vs. 4.51; p<0.001) and pertussis agglutinins (51.93 vs. 23.34; p=0.008) was, in all cases significantly higher in the combined vaccine group when compared to the separate administration group respectively (See Table 2). In the cases of diphtheria and pertussis, this significance was lost when comparing the ratio of response to baseline titer. A subset of sera was analyzed for antibody to specific pertussis antigens: pertussis toxin (PT), FHA and 69K protein. The response to PT (85.92 vs. 17.60; p=0.001) and FHA (2.32 vs. 0.79; p=0.0001) was significantly higher in the combined vaccine group compared to the separate administration group, respectively (See Table 3). However, no difference was observed for 69K (combined vaccine 66.43 vs. separate administration 41.55; p=0.11).
c. Large Scale Infant Safety Study with Immunogenicity Subset
   A large scale safety study in infants was conducted at the Kaiser Permanente Medical Care Program of Northern California. This study employed both a non-randomized and a randomized design. The former allowed for accumulation of data for the combined vaccine in a large number of subjects (n=5777). The latter provided a controlled study whereby the safety of the combined vaccine (n=720) could be prospectively compared with a cohort receiving DTP-Hib conjugate (n=691) concurrently as separate injections. Thus, a total of 6497 subjects received 3 doses of the combined vaccine (D,T,P, and Hib conjugate combined) at approximately 2, 4 and 6 months of age.
   The incidence of local and systemic events occurring within 24 hours of immunization was determined. Rare events, such as seizures, were assessed by evaluating hospitalizations within 60 days and emergency room utilization within 30 days of immunization. For the three visit series, local and systemic reactions did not occur consistently more frequently following the administration of the combined vaccine when compared to DTP alone. In addition, there was no significant difference for rates of medical adverse events observed in hospitalization or emergency room utilizations. The SIDS death rate was somewhat lower in the combined vaccine cohort than in the control cohort.
   The antibody responses to Hib polysaccharide, diphtheria, tetanus and pertussis were measured one month following the third dose of the combined vaccine in a subset of 123 subjects. The values obtained were comparable to those derived from the multicenter safety and immunogenicity study in infants described above (See Table 4).
d. Serological Procedures
   Serological assays were performed by ELISA for the Haemophilus influenzae type b polysaccharide and tetanus (for the infant studies) antibodies, by the Vero cell assay (J. Biol. Stand. 2:189-201; J. Biol. Stand. 2:203-209) for diphtheria antibodies, by the mouse lethal method (Bull WHO 43:453-459; Minimum Requirements: Tetanus Toxoid Appendix A, June 15, 1953, Pub. Health Serv., NIH; Craig, 1986, Manual of Clinical Laboratory Immunol., pp 408-414, 3rd ed.) for the tetanus antibodies (toddler study), and by the microagglutination test (Manclark et al, 1986, Manual of Clinical Laboratory Immunol., pp 388-394, 3rd ed.), for pertussis antibodies.

As will be appreciated from the clinical studies reported herein, the response by infants to each of the vaccine components was higher in the case of the combined vaccine than for the concurrent but separate administration of the DPT and Hib conjugate vaccines. The use of such a combined vaccine thus can reduce the number of immunizations given to infants by half and is an important step toward improving vaccine delivery.

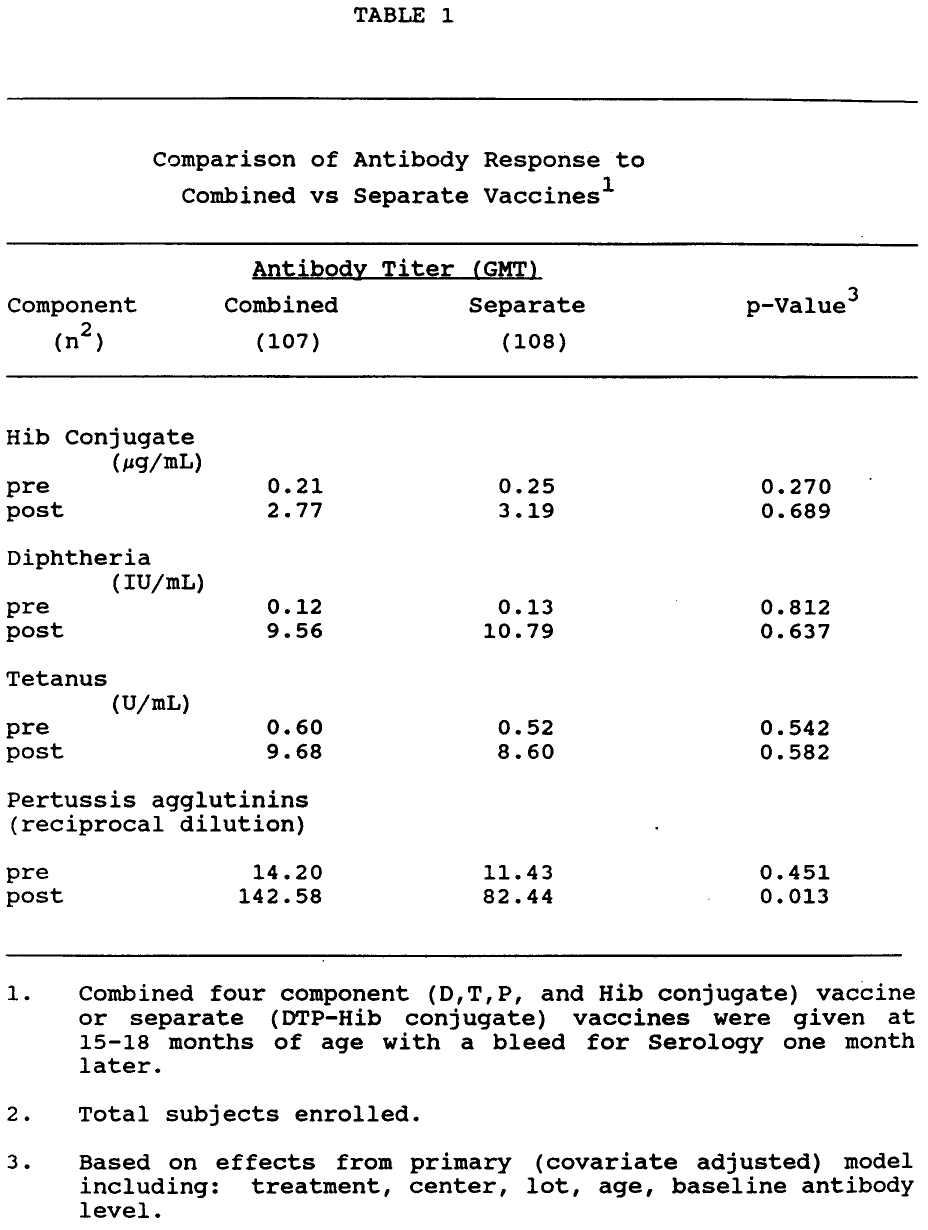

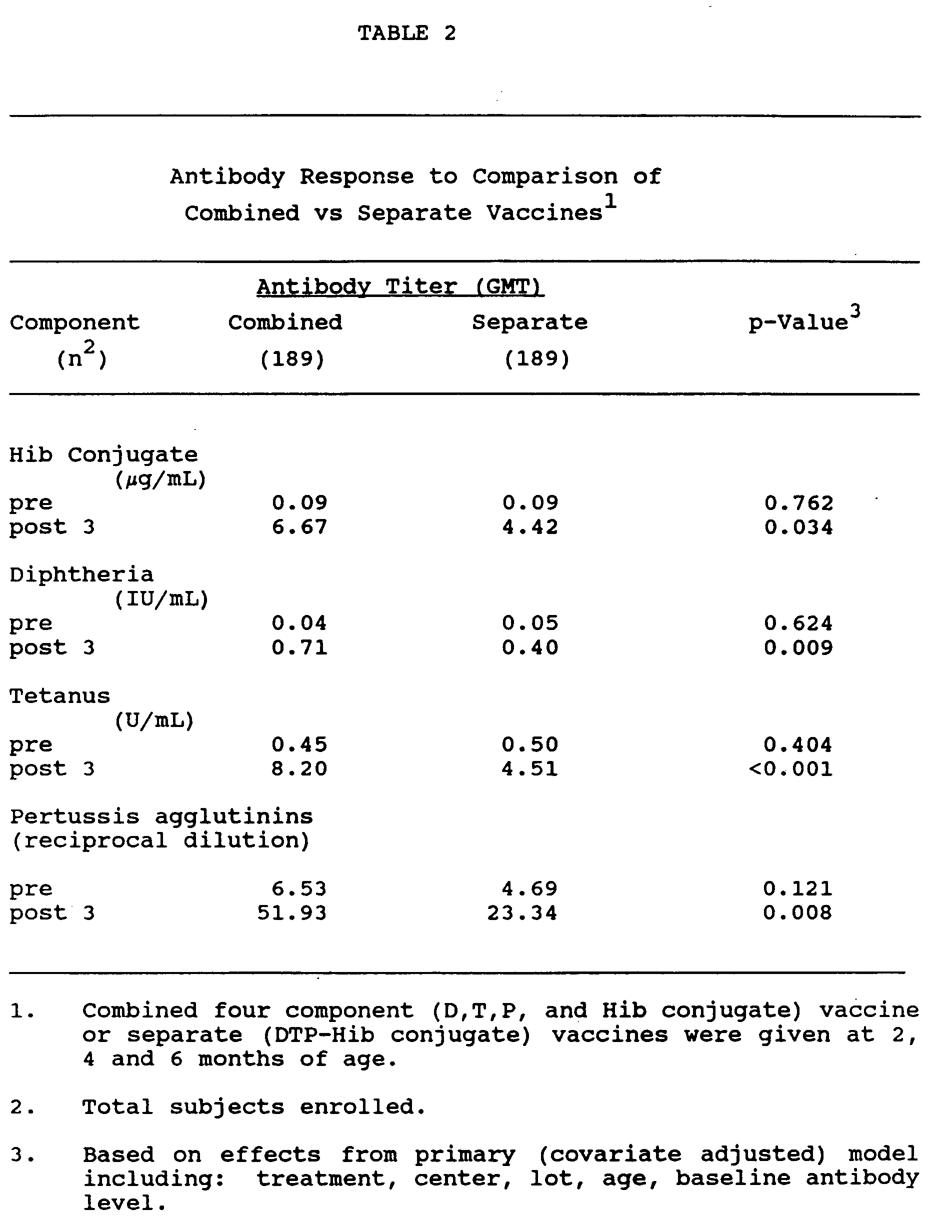

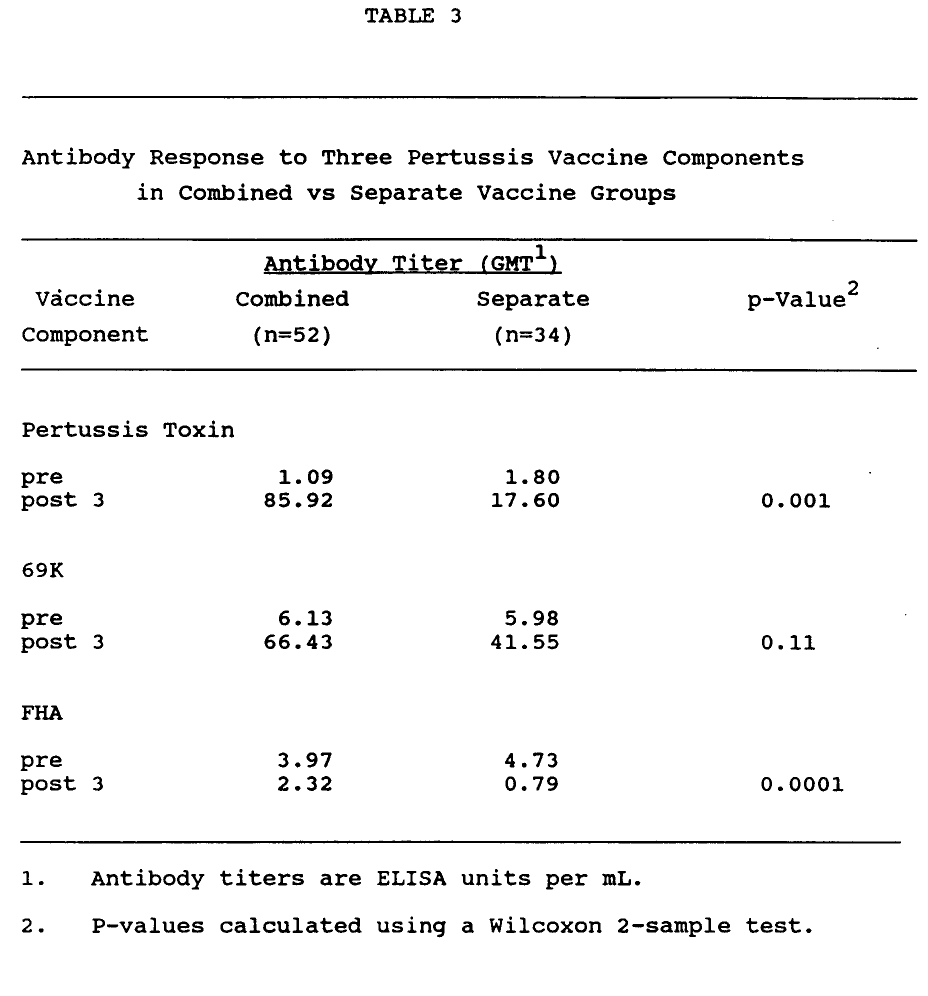

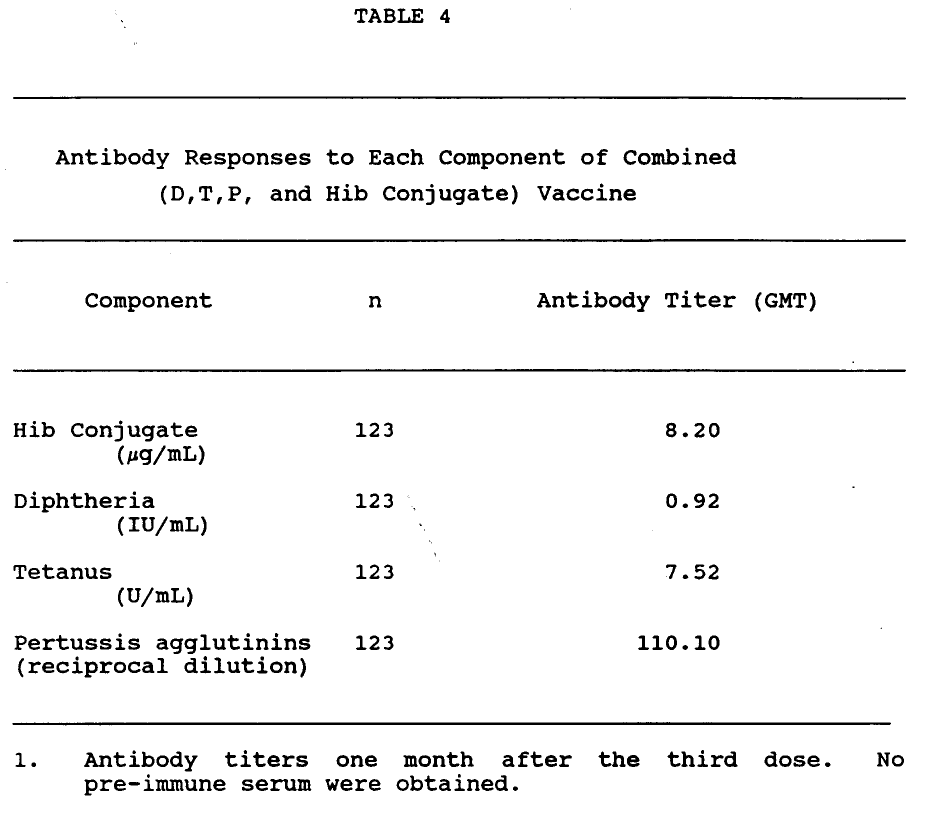

## Claims

1. A combined pediatric vaccine composition comprising, in a single immunizing dose, a mixture of diphtheria, tetanus and pertussis antigens and a conjugate of fragments of the capsular polysaccharide antigen of Haemophilus influenzae type b and CRM₁₉₇ protein in a pharmaceutically acceptable aqueous vehicle, said combined vaccine composition providing an enhanced antibody response by infants to each of the diphtheria, tetanus, pertussis and Haemophilus influenzae type b vaccine components.

2. A vaccine composition as claimed in claim 1 wherein the diphtheria antigen is diphtheria toxoid, the tetanus antigen is tetanus toxoid and the pertussis antigen is inactivated Bordetella pertussis cells.

3. A vaccine composition as claimed in claim 2 wherein the diphtheria toxoid is aluminum phosphate adsorbed, the tetanus toxoid is aluminum phosphate adsorbed, the inactivated Bordetella pertussis cells are suspended in a solution comprising potassium phosphate monobasic, sodium phosphate dibasic and sodium chloride.

4. A vaccine composition as claimed in any one of claims 1 to 3 wherein it is adapted for intramuscular administration.

5. a) diphtheria antigens,
b) tetanus antigens,
c) pertussis antigens, and
d) a conjugate of fragments of the capsular polysaccharide antigen of Haemophilus influenzae type b and CRM₁₉₇ protein
in combination for use as a vaccine for infants.

6. Use of
a) diphtheria antigens,
b) tetanus antigens,
c) pertussis antigens, and
d) a conjugate of fragments of the capsular polysaccharide antigen of Haemophilus influenzae type b and CRM₁₉₇ protein
in combination for the manufacture of a vaccine for the vaccination of infants.

## Patentansprüche

1. Pädriatische Kombinationsvakzin-Zusammensetzung, umfassend in einer einzelnen immunisierenden Dosis eine Mischung von Diphtherie-, Tetanus- und Keuchhusten-Antigenen und ein Konjugat von Fragmenten des Kapsel-Polysaccharid-Antigens von Haemophilus influenzae Typ b und CRM₁₉₇-Protein in einem pharmazeutisch annehmbaren wäßrigen Vehikel, wobei die Kombinationsvakzin-Zusammensetzung eine verstärkte Antikörperantwort durch Kleinkinder auf jede Diphtherie-, Tetanus-, Keuchhusten- und Haemophilus influenzae Typ b-Vakzinkomponente bringt.

2. Vakzin-Zusammensetzung gemäß Anspruch 1, worin das Diphtherieantigen Diphtherietoxoid ist, das Tetanusantigen Tetanustoxoid ist und das Keuchhustenantigen inaktivierte Bordetella pertussis-Zellen sind.

3. Vakzin-Zusammensetzung gemäß Anspruch 2, worin das Diphtherietoxoid Aluminiumphosphat-adsorbiert ist, das Tetanustoxoid Aluminiumphosphat-adsorbiert ist, die inaktivierten Bordetella pertussis-Zellen in einer Lösung suspendiert sind, die einbasisches Kaliumphosphat, zweibasisches Natriumphosphat und Natriumchlorid umfaßt.

4. Vakzin-Zusammensetzung gemäß einem der Ansprüche 1 bis 3, wobei diese für die intramuskuläre Verabreichung adaptiert ist.

5. a) Diphtherieantigene, b) Tetanusantigene, c) Keuchhustenantigene und d) ein Konjugat von Fragmenten des Kapsel-Polysaccharid-Antigens von Haemophilus influenzae Typ b und CRM₁₉₇-Protein, in Kombination zur Verwendung als Vakzin für Kleinkinder.

6. Verwendung von a) Diphtherieantigenen, b) Tetanusantigenen, c) Keuchhustenantigenen und d) einem Konjugat von Fragmenten des Kapsel-Polysaccharid-Antigens von Haemophilus influenzae Typ b und CRM₁₉₇-Protein, in Kombination zur Herstellung eines Vakzins zum Impfen von Kleinkindern.

## Revendications

1. Composition de vaccin pédiatrique combiné comprenant, en une dose d'immunisation unique, un mélange d'antigènes de la diphtérie, du tétanos et de la coqueluche, et un conjugué de fragments de l'antigène polysaccharide capsulaire d'Haemophilus influenzae type b et de la protéine CRM₁₉₇ dans un excipient aqueux pharmaceutiquement acceptable, ladite composition de vaccin combiné fournissant une réaction d'anticorps améliorée chez les nourrissons pour chacun des composants du vaccin contre la diphtérie, le tétanos, la coqueluche et d'Haemophilus influenzae type b.

2. Composition de vaccin suivant la revendication 1, dans laquelle l'antigène de la diphtérie est l'anatoxine diphtérique, l'antigène du tétanos est l'anatoxine tétanique et l'antigène de la coqueluche est composé de cellules inactivées de Bordetella pertussis.

3. Composition de vaccin suivant la revendication 2, dans laquelle l'anatoxine diphtérique est adsorbée sur du phosphate d'aluminium, l'anatoxine tétanique est adsorbée sur du phosphate d'aluminium et les cellules inactivées de Bordetella pertussis sont en suspension dans une solution contenant du phosphate de potassium monobasique, du phosphate de sodium dibasique et du chlorure de sodium.

4. Composition de vaccin suivant l'une quelconque des revendications 1 à 3, qui est adaptée a une administration intramusculaire.

5. a) Antigènes de la diphtérie,
b) antigènes du tétanos,
c) antigènes de la coqueluche, et
d) un conjugué de fragments de l'antigène polysaccharide capsulaire d'Haemophilus influenzae type b et de protéine CRM₁₉₇
combinés pour une utilisation comme un vaccin pour les nourrissons.

6. Utilisation
a) d'antigènes de la diphtérie,
b) d'antigènes du tétanos,
c) d'antigènes de la coqueluche, et
d) d'un conjugué de fragments de l'antigène polysaccharide capsulaire d'Haemophilus influenzae type b et de protéine CRM₁₉₇
combinés pour la fabrication d'un vaccin pour la vaccination des nourrissons.
